# FASCICULE DE BREVET EUROPEEN

(11) **EP 2 231 131 B1**
(45) Date de publication et mention de la délivrance du brevet: **19.01.2011**
(21) Numéro de dépôt: 08840675.6
(22) Date de dépôt: 07.10.2008
(51) Int. Cl.: A61K 9/51, A61K 31/282, A61K 47/48, C08F 290/06

(54) **FORMULATIONS DE COMPOSES ORGANOPLATINIQUES EN PRESENCE DE POLYMERES ASSOCIATIFS, PRODUITS OBTENUS ET LEURS UTILISATIONS**
FORMULIERUNGEN VON ORGANISCHEN PLATINVERBINDUNGEN IN ANWESENHEIT VON ASSOZIATIVEN POLYMEREN, SO ERHALTENE PRODUKTE UND IHRE ANWENDUNGEN
FORMULATIONS OF ORGANO-PLATINIC COMPOUNDS IN THE PRESENCE OF ASSOCIATIVE POLYMERS, PRODUCTS THUS OBTAINED AND USES THEREOF

(30) Priorité: 19.10.2007 FR 0707306
(43) Date de publication de la demande: 29.09.2010
(73) Titulaire: COATEX S.A.S., 69730 Genay (FR)
(72) Inventeur: GUERRET, Olivier, F-69890 La Tour de Salvagny (FR); SUAU, Jean-Marc, F-69480 Lucenay (FR)
(74) Mandataire: Tetaz, Franck Claude Edouard
(86) Numéro de dépôt international: PCT/IB2008/002669
(87) Numéro de publication internationale: WO 2009/050555

(56) Documents cités:
- EP-A- 0 350 414
- EP-A- 1 329 221
- WO-A-03/037383
- WO-A-2004/044022
- WO-A-2004/112757
- US-A1- 2005 208 136

## Description

L'invention concerne un nouveau type de formulation de composés organoplatiniques (dérivés organiques du platine) pour administration par voie orale.

Parmi les composés organoplatiniques, le cisplatine et l'oxaliplatine sont des molécules à base de platine utilisées dans le traitement de différents cancers tels les sarcomes, carcinomes (cancer du poumon à petites cellules, cancer de l'ovaire...), lymphomes. Ils appartiennent à la classe des composés alkylants l'ADN avec le carboplatine.

Or, ces composés provoquent de nombreux effets indésirables, notamment lorsqu'ils sont administrés par voie intraveineuse : nephrotoxicité gastrointestinale (nausées, vomissements), neurotoxicité et myélosuppression modérée. Des travaux ont été réalisés en vue de tester des analogues de ces principes actifs, mais aucun ne s'est révélé aussi efficace que les molécules de base. On a également mené des études en vue de combiner d'autres thérapies, avec des quantités réduites de cisplatine et d'oxaliplatine, mais on a alors diminué l'efficacité du traitement.

Un autre axe de recherche concerne les méthodes d'administration des composés organoplatiniques par voie orale. L'enjeu consiste ici à mettre au point un procédé pour limiter la toxicité de ces principes actifs, de leur permettre de franchir la barrière gastrointestinale, de contrôler leur libération au cours de leur trajet, et enfin d'offrir au patient la possibilité d'un traitement par voie orale qu'il peut lui-même s'administrer.

Dans cette lignée, la Demande de Brevet FR 2 759 293 décrit un procédé assez complexe de fabrication de microgranules contenant du cisplatine. Il comprend en effet une étape de fixation du cisplatine sur les microgranules par pulvérisation d'une solution organique contenant du cisplatine sur un support neutre, puis une seconde étape d'enrobage à l'aide de polymères qui permettent la libération prolongée du principe actif. Les substances d'enrobage sont choisies parmi les polymères cellulosiques ou méthacryliques, et préférentiellement parmi les copolymères de l'acide méthacrylique avec un ester acrylique commercialisés sous la marque Eudragit™. Cet enrobage constitue un vernis protégeant le principe actif, qui va se dissoudre progressivement en milieu aqueux : on régule ainsi la libération du composé organoplatinique de manière continue.

Poursuivant ses recherches pour enrichir l'état de la technique avec une solution alternative permettant d'administrer des composés organoplatiniques par voie orale, tout en protégeant le patient de leur toxicité, en permettant le passage de la barrière gastrointestinale et en contrôlant la libération progressive du principe actif, la Demanderesse a mis au point de nouvelles formulations, qui contiennent au moins un composé organoplatinique et au moins un polymère hydrosoluble associatif, ces formulations étant **caractérisées en ce que** ledit polymère hydrosoluble associatif est le résultat de la polymérisation :
- de l'acide (méth)acrylique,
- d'au moins un monomère non hydrosoluble, qui est préférentiellement un ester (méth)acrylique choisi très préférentiellement parmi l'acrylate d'éthyle, l'acrylate de butyle, le méthacrylate de méthyle et leurs mélanges,
- et d'au moins un monomère de formule (I) : 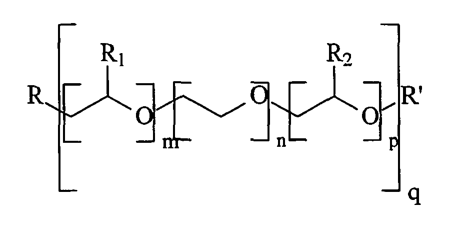
- m, n, p et q sont des entiers et m, n, p sont inférieurs à 150 et l'un d'eux au moins est supérieur à 0, préférentiellement 20,
- R comporte une fonction vinylique polymérisable,
- R₁ et R₂ sont identiques ou différents et représentent des atomes d'hydrogène ou des groupements alkyls ayant de 1 à 4 atomes de carbone,
- R' est un groupement hydrophobe ayant de 14 inclus à 32 inclus atomes de carbone, et est préférentiellement choisi parmi les groupements alkyles ramifiés ayant de 14 inclus à 32 inclus atomes de carbone, et parmi les groupements aromatiques substitués ayant de 14 inclus à 32 inclus atomes de carbone.

Les polymères hydrosolubles associatifs décrits ci-dessus, sont encore appelés polymères de type HASE (acronyme anglo-saxon pour Hydrophobically Alkali Swellable Emulsion) : ils désignent des polymères acryliques à base d'acide (méth)acrylique, d'un monomère non hydrosoluble qui est préférentiellement un ester (méth)acrylique et d'un monomère hydrophobe associatif. Ils se distinguent des polymères de type ASE (Alkali Soluble Emulsion) tel que l'Eudragit™ par le fait qu'ils contiennent un monomère hydrophobe susceptible de développer des interactions associatives dans certaines conditions de pH.

Ce monomère possède la propriété, en milieu aqueux et à pH élevé, de développer des interactions associatives qui conduisent à la formation de nodules nanométriques (diamètre compris entre 0,1 et 100 nm) : ce sont autant de cages de solvatation vis-à-vis des composés organoplatiniques. Le principe actif ainsi isolé va être relargué progressivement à travers les nodules créés par le polymère. Le patient est donc protégé et l'efficacité du traitement est améliorée.

Un des mérites de la Demanderesse est d'avoir su utiliser ce mécanisme associatif pour isoler les composés organoplatiniques. Un autre de ses mérites est d'avoir su sélectionner et mettre au point les monomères hydrophobes particuliers, qui permettent d'isoler efficacement les composés organoplatiniques : cette sélection repose notamment sur la longueur et la nature de la chaîne R' telle qu'indiqué plus haut, et comme illustré dans les exemples de la présente Demande.

Dans une première variante, les formulations selon l'invention sont des formulations aqueuses qui contiennent le composé organoplatinique et le polymère hydrosoluble associatif ; elles sont obtenues par mélange des différents constituants, et régulation du pH à une valeur supérieure à 6, de manière à déclencher l'effet associatif induit par le monomère de formule (I). Pratiquement, le pH est ajusté au moyen d'une base minérale ou organique et les constituants (composé organoplatinique, eau, polymère, ainsi que la base minérale ou organique) sont introduits sous agitation dans un réacteur. Cette variante correspond à la forme liquide du produit que le patient peut ingérer : celle d'un sirop.

Dans une deuxième variante, on abaisse le pH des formulations précédemment obtenues à une valeur inférieure à 6, préférentiellement 3, par exemple au moyen d'un acide moyennement fort à fort : à travers un mécanisme de précipitation, la structure des nodules "s'effondre" sur les composés organoplatiniques. Grâce à une étape ultérieure d'élimination de l'eau, tel que par exemple séchage ou filtration, on obtient des formulations sèches qui contiennent le composé organoplatinique et le polymère associatif. Cette variante correspond à la forme sèche du produit que le patient peut avaler : celle d'un granulé.

Aussi, un autre intérêt de l'invention réside dans l'existence de ces deux variantes qui conduisent chacune à une forme particulière du produit final, qui sont précisément les deux formes recherchées par le galéniste. En outre, l'unité d'invention est assurée entre ces formes par la présence systématique du polymère hydrosoluble associatif et du composé organoplatinique.

Un premier objet de l'invention consiste donc en des formulations aqueuses, contenant au moins un composé organoplatinique et au moins un polymère hydrosoluble associatif, et **caractérisées en ce que** ledit polymère est le résultat de la polymérisation :
- de l'acide (méth)acrylique,
- d'au moins un monomère non hydrosoluble, qui est préférentiellement un ester (méth)acrylique choisi très préférentiellement parmi l'acrylate d'éthyle, l'acrylate de butyle, le méthacrylate de méthyle et leurs mélanges,
- et d'au moins un monomère de formule (I) : 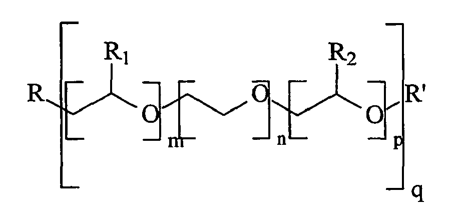
- m, n, p et q sont des entiers et m, n, p sont inférieurs à 150 et l'un d'eux au moins est supérieur à 0, préférentiellement 20,
- R comporte une fonction vinylique polymérisable,
- R₁ et R₂, sont identiques ou différents et représentent des atomes d'hydrogène ou des groupements alkyls ayant de 1 à 4 atomes de carbone,
- R' est un groupement hydrophobe ayant de 14 inclus à 32 inclus atomes de carbone, et est préférentiellement choisi parmi les groupements alkyles ramifiés ayant de 14 inclus à 32 inclus atomes de carbone, et parmi les groupements aromatiques substitués ayant de 14 inclus à 32 inclus atomes de carbone.

Il est entendu que ces 3 monomères sont indispensables dans la constitution des polymères hydrosolubles associatifs selon l'invention. L'homme du métier pourra ajouter d'autres monomères, tel que par exemple un monomère possédant aux moins deux fonctions insaturées éthyléniques, encore appelé monomère réticulant.

Dans une première variante, ces formulations aqueuses sont aussi **caractérisées en ce que** leur pH est supérieur à 6, préférentiellement 6,5, très préférentiellement 7.

Dans une deuxième variante, ces formulations aqueuses sont aussi **caractérisées en ce que** leur pH est inférieur à 6, préférentiellement 3. Il est évident que cette variante est obtenue par diminution du pH, le pH ayant été préalablement augmenté à une valeur supérieure à 6 de manière à déclencher l'effet associatif qui permet de protéger le composé organoplatinique.

Ces formulations aqueuses sont aussi **caractérisées en ce qu**'elles contiennent de 0,1 % à 30 %, préférentiellement de 5 % à 30 %, très préférentiellement de 10 % à 30 % en poids sec dudit composé organoplatinique, par rapport au poids sec dudit polymère hydrosoluble associatif.

Ces formulations aqueuses sont aussi **caractérisées en ce qu**'elles contiennent de 0,1 % à 15 %, préférentiellement de 1 % à 10 % en poids de matière sèche par rapport à leur poids total.

Ces formulations aqueuses sont aussi **caractérisées en ce que** le composé organoplatinique est choisi parmi le carboplatine, l'oxaliplatine et leurs mélanges, et est préférentiellement l' oxaliplatine.

Ces formulations aqueuses sont aussi **caractérisées en ce qu**'elles contiennent éventuellement au moins un autre agent anticancéreux choisi parmi le fluoro-uracil, le S1, l'association de la vinblastine avec la bléomycine, l'association de l'étoposide avec la bléomycine ou le paclitaxel.

Un autre objet de l'invention consiste en des granulés contenant au moins un composé organoplatinique et au moins un polymère hydrosoluble associatif, et **caractérisés en ce que** ledit polymère est le résultat de la polymérisation :
- de l'acide (méth)acrylique,
- d'au moins un monomère non hydrosoluble, qui est préférentiellement un ester (méth)acrylique choisi très préférentiellement parmi l'acrylate d'éthyle, l'acrylate de butyle, le méthacrylate de méthyle et leurs mélanges,
- et d'au moins un monomère de formule (I) : 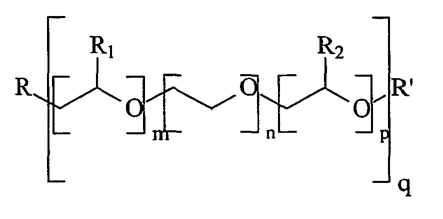
- m, n, p et q sont des entiers et m, n, p sont inférieurs à 150 et l'un d'eux au moins est supérieur à 0, préférentiellement 20,
- R comporte une fonction vinylique polymérisable,
- R₁ et R₂ sont identiques ou différents et représentent des atomes d'hydrogène ou des groupements alkyls ayant de 1 à 4 atomes de carbone,
- R' est un groupement hydrophobe ayant de 14 inclus à 32 inclus atomes de carbone, et est préférentiellement choisi parmi les groupements alkyles ramifiés ayant de 14 inclus à 32 inclus atomes de carbone, et parmi les groupements aromatiques substitués ayant de 14 inclus à 32 inclus atomes de carbone.

Ces granulés sont aussi **caractérisés en ce qu**'ils contiennent moins de 5 %, préférentiellement 1 %, très préférentiellement 0,1 % en poids d'eau, tel que mesuré par pesée différentielle après évaporation dans une étuve à 110°C pendant 1 heure.

Ces granulés sont aussi **caractérisés en ce qu**'ils contiennent de 0,1 % à 30 %, préférentiellement de 5 % à 30 %, très préférentiellement de 10 % à 30 % en poids sec dudit composé organoplatinique, par rapport au poids sec dudit polymère hydrosoluble associatif.

Ces granulés sont aussi **caractérisés en ce que** le composé organoplatinique est choisi parmi le carboplatine, l'oxaliplatine et leurs mélanges, et est préférentiellement l' oxaliplatine.

Ces granulés sont aussi **caractérisés en ce qu**'ils contiennent éventuellement au moins un autre agent anticancéreux choisi parmi le fluoro-uracil, le S1, l'association de la vinblastine avec la bléomycine, l'association de l'étoposide avec la bléomycine ou le paclitaxel.

Ces granulés sont aussi **caractérisés en ce qu**'ils contiennent un agent d'enrobage qui est choisi parmi un polymère cellulosique, un copolymère de l'acide (méth)acrylique avec un ester acrylique, et leurs mélanges.

Ces granulés sont aussi **caractérisés en ce qu**'ils contiennent un agent lubrifiant qui est préférentiellement le talc.

Ces granulés sont aussi **caractérisés en ce qu**'ils contiennent un agent plastifiant qui est préférentiellement le triéthyle citrate.

Ces granulés sont aussi **caractérisés en ce qu**'ils contiennent un agent tensioactif qui est préférentiellement le polysorbate 80 (connu aussi sous le nom de Tween™ 80, et commercialisé par la société UNIQEMA™).

Un autre objet de l'invention est une préparation pharmaceutique **caractérisée en ce qu**'elle contient une formulation aqueuse selon l'invention ou un granulé selon l'invention.

Un dernier objet de l'invention est l'utilisation d'une formulation aqueuse selon l'invention et d'un granulé selon l'invention, pour fabriquer un médicament à administration par voie orale, et destiné à être mis en oeuvre en polychimiothérapie.

### EXEMPLES

### Exemple 1 : Synthèse de monomères mis en oeuvre selon l'invention:

### Protocole a : Synthèse de monomère méthacrylique :

Dans un réacteur de 1 litre, on pèse :
- 400 grammes de l'alcool béhénique condensé avec 25 moles d'oxyde d'éthylène fondu,
- 0,0994 gramme d'alloocimène,
- 43,75 grammes d'anhydride méthacrylique.

On chauffe le mélange à 82°C ± 2°C et on poursuit la cuisson pendant 3 heures à cette température. Le macromonomère obtenu est alors dilué avec 396 g d'acide méthacrylique afin d'obtenir une solution liquide à température ambiante.

### Protocole b : Synthèse de monomère uréthanne :

Dans une première étape, on réalise un précondensat en pesant dans un erlen :
- 13,726 grammes de toluène di isocyanate,
- 36,1 grammes d'acrylate d'éthyle,
- 0,077 gramme d'alloocimène,
- 0,198 gramme de dilaurate de dibuthyle d'étain.

On pèse ensuite dans une ampoule de coulée 10,257 grammes de méthacrylate d'éthylène glycol et 10 grammes d'acrylate d'éthyle. On coule le contenu de cette ampoule dans l'erlen en 20 minutes à une température inférieure à 35°C, et on laisse réagir 30 minutes.

Dans une deuxième étape, on réalise la condensation en pesant 132 grammes de tri styryl phénol condensé avec 25 moles d'oxyde d'éthylène dans un réacteur de 1 000 ml que l'on maintiendra fondu à 65°C. On coule alors le précondensat en 20 minutes à 65°C sur cet alcool puis on cuit 2 heures à 65°C. Enfin, on dilue le mélange avec 15,5 grammes d'acrylate d'éthyle et 84,6 grammes d'eau bi permutée afin d'obtenir un liquide à température ambiante.

### Protocole c : Synthèse de monomère hémimaléate :

Dans un réacteur de 1 litre on pèse :
   - 400 grammes de l'alcool ramifié de 32 atomes de carbone condensé avec 25 moles d'oxyde d'éthylène fondu,
   - 0,0994 gramme d'alloocimène,
   - 25,3 grammes d'anhydride maléique.

On chauffe le mélange à 822°C ± 2°C et on poursuit la cuisson pendant 3 heures. Le macromonomère alors obtenu est dilué avec 425 g d'acide méthacrylique afin d'obtenir une solution liquide à température ambiante.

### Exemple 2 : synthèse de polymères hydrosolubles associatifs :

### Protocole A :

Dans un réacteur de 1 litre on pèse 280 grammes d'eau bi permutée et 3,89 grammes de dodecyl sulfate de sodium. On chauffe en pied de cuve à 82°C ± 2°C.

Pendant ce temps, on prépare une pré émulsion, en pesant dans un bécher :
- 112,4 grammes d'eau bi permutée,
- 2,1 grammes de dodecyl sulfate de sodium,
- 80,6 grammes d'acide méthacrylique,
- 146,1 grammes d'acrylate d'éthyle,
- 55,6 grammes d'une solution de macromonomère telle que décrite dans le protocole a).

On pèse ensuite 0,85 gramme de persulfate d'ammonium dilués dans 10 grammes d'eau bi permutée pour le premier catalyseur, et 0,085 gramme de métabisulfite de sodium dilués dans 10 grammes d'eau bi permutée pour le second catalyseur. Lorsque le pied de cuve est à température, on ajoute les 2 catalyseurs et on effectue la polymérisation pendant 2 heures à 76°C ± 2°C, avec ajout en parallèle de la pré émulsion. On rince la pompe avec 20 grammes d'eau bi permutée et on cuit 1 heure à 76°C ± 2°C. On refroidit enfin à température ambiante et on filtre le polymère ainsi obtenu.

### Protocole B :

Dans un réacteur de 1 litre on pèse 280 grammes d'eau bi permutée et 3,89 grammes de dodecyl sulfate de sodium. On chauffe en pied de cuve à 82°C ± 2°C.

Pendant ce temps, on prépare une pré émulsion, en pesant dans un bécher:
- 334 grammes d'eau bi permutée,
- 3,89 grammes de dodecyl sulfate de sodium,
- 0,92 gramme de dodecyl mercaptan,
- 80,6 grammes d'acide méthacrylique,
- 160,55 grammes d'acrylate d'éthyle,
- 60,4 grammes de la solution de méthacryluréthanne décrite dans le protocole b).

On pèse ensuite 0,33 gramme de persulfate d'ammonium dilué dans 10 grammes d'eau bi permutée pour le premier catalyseur, et 0,28 gramme de métabisulfite de sodium dilués dans 10 grammes d'eau bi permutée pour le second catalyseur. Lorsque le pied de cuve est à température, on ajoute les 2 catalyseurs et on effectue la polymérisation pendant 2 heures à 84°C ± 2°C, avec ajout en parallèle de la pré émulsion. On rince la pompe avec 20 grammes d'eau bi permutée et on cuit 1 heure à 84°C ± 2°C. On refroidit enfin à température ambiante et on filtre.

### Protocole C :

Ce protocole est identique au protocole B, à la différence près qu'on supprime ici le dodecyl mercaptan dans la première étape de pesée.

### Protocole D :

Ce protocole est identique au protocole A, à la différence près qu'on introduit 0,9 gramme de dodecyl mercaptan dans l'étape de pesée initiale dans le bécher.

### Exemple 3: fabrication de formulations aqueuses de polymères hydrosolubles associatifs en présence d'oxaliplatine, à un pH supérieur à 6,5.

Dans tous les essais qui suivent l'oxaliplatine est le produit du même nom, commercialisé par la société MIDAS PHARMA™.

### Premier essai témoin :

Cet essai est un témoin qui consiste à introduire dans l'eau de l'oxaliplatine, sans polymère hydrosoluble associatif.

On introduit 0,09 g d'oxaliplatine dans 8,6 grammes d'eau, le pH étant ajusté à une valeur égale à 7 au moyen d'une solution de soude à 5 %. La concentration d'oxaliplatine dans l'eau est ici égale à 10,5 g/L, alors que la solubilité de l'oxaliplatine est inférieure à 7,9 grammes par litre d'eau.

On obtient une poudre en suspension dans l'eau : pour la concentration étudiée, l'oxaliplatine se disperse mais n'est qu'en partie soluble dans l'eau.

### Deuxième essai témoin :

Cet essai est un témoin qui consiste à introduire dans l'eau de l'oxaliplatine avec un polymère hydrosoluble non associatif.

On introduit 0,09 g d'oxaliplatine dans 8,6 grammes d'eau, le pH étant ajusté à une valeur égale à 8 au moyen d'une solution de soude à 5 %. La concentration d'oxaliplatine dans l'eau est ici égale à 10,5 g/L, alors que la solubilité de l'oxaliplatine est inférieure à 7,9 grammes par litre d'eau.

On ajoute alors 1,9 g d'une émulsion de polymère hydrosoluble à 30 % d'extrait sec, ledit polymère étant constitué à 36,9 % en poids d'acide méthacrylique et 63,1 % en poids d'acrylate d'éthyle.

On obtient une poudre en suspension dans l'eau : le polymère n'est pas parvenu à encapsuler l'oxaliplatine.

### Essais n° 1 à 12 :

Ces essais illustrent des formulations aqueuses contenant de l'oxaliplatine et des polymères hydrosolubles associatifs possédant :
- un monomère (méth)acrylique,
- un monomère non hydrosoluble,
- et un monomère hydrophobe associatif.

On pèse exactement 1,9 g d'une émulsion de polymère associatif hydrosoluble à 30 % d'extrait sec. On ajoute 8,6 grammes d'une solution d'eau bipermutée et enfin au minimum 100 mg d'oxaliplatine soit une concentration minimale de 0,86 % d'oxaliplatine dans la formulation. Le milieu est agité pendant cette addition, et le pH est ajusté à 8 à partir d'une solution de soude à 5 %

Dans le tableau 1, on a indiqué :
- le % en poids sec pesé d'oxaliplatine par rapport au poids sec du polymère associatif mis en oeuvre,
- le pourcentage en poids de matière sèche de la formulation réalisée (ce calcul prend en compte la quantité de soude ajoutée dans le milieu pour réguler le pH à 8),
- l'aspect de la formulation obtenue.

**Tableau 1**

| Essai n° | % oxaliplatine / polymère sec | % matière sèche / formulation totale | Aspect de la formulation |
|---|---|---|---|
| 1 | 14,8 | 5,2 | Poudre en dispersion dans l'eau |
| 2 | 14,8 | 5,2 | Poudre en dispersion dans l'eau |
| 3 | 16,3 | 5,4 | Solution |
| 4 | 13,2 | 5,8 | Solution |
| 5 | 13,6 | 5,7 | Solution |
| 6 | 14,5 | 5.8 | Solution |
| 7 | 14,7 | 5,8 | Solution |
| 8 | 13,8 | 5,8 | Solution |
| 9 | 14,4 | 5,7 | Solution |
| 10 | 15,0 | 5,8 | Solution |
| 11 | 15,0 | 5,8 | Solution |
| 12 | 13,5 | 5,7 | Solution |

Dans chacun des essais n° 1 à 12, on renvoie aux protocoles A, B ou C pour la synthèse du polymère : en fonction des ratios monomériques visés, l'homme du métier calcule les poids des différents constituants à peser dans chacun de ces protocoles.

De même, on renvoie aux protocoles a, b ou c pour la synthèse du monomère de formule (I) et on précise la nature et la masse de l'alcool mis en oeuvre.

L'essai 1 met en oeuvre un polymère hydrosoluble associatif, **caractérisé en ce qu**'il est obtenu par réaction (selon le protocole B) entre :
- 36,5 % en poids d'acide méthacrylique,
- 54,1 % en poids d'acrylate d'éthyle,
- 9,4 % en poids d'un monomère de formule (I) (selon le protocole b) dans lequel on pèse 100 grammes de l'alcool laurique condensé avec 25 moles d'oxyde d'éthylène) avec R qui est le résultat de la condensation entre le méthacrylate d'éthylène glycol et le toluène diisocyanate, m = p = 0, q = 1, n = 25 et R' est un groupement alkyle contenant 12 atomes de carbone.

L'essai 2 met en oeuvre un polymère hydrosoluble associatif, **caractérisé en ce qu**'il est obtenu par réaction (selon le protocole A) entre :
- 36,0 % en poids d'acide méthacrylique,
- 54,5 % en poids d'acrylate d'éthyle,
- 9,5 % en poids d'un monomère de formule (I) (selon le protocole a) dans lequel on pèse 346,6 grammes de l'alcool dodécylique condensé avec 25 moles d'oxyde d'éthylène) avec R qui est le méthacrylate, m = p = 0, q = 1, n = 25 et R' est un groupement alkyle contenant 10 atomes de carbone.

L'essai 3 met en oeuvre un polymère hydrosoluble associatif, **caractérisé en ce qu**'il est obtenu par réaction (selon le protocole A) entre :
- 36,7 % en poids d'acide méthacrylique,
- 53,1 % en poids d'acrylate d'éthyle,
- 10,2 % en poids d'un monomère de formule (I) (selon le protocole a) avec R qui est le méthacrylate, m = p = 0, q = 1, n = 25 et R' est un groupement alkyle contenant 22 atomes de carbone.

L'essai 4 met en oeuvre un polymère hydrosoluble associatif, **caractérisé en ce qu**'il est obtenu par réaction (selon le protocole C) entre :
- 38,9 % en poids d'acide méthacrylique,
- 53,1 % en poids d'acrylate d'éthyle,
- 8,0 % en poids d'un monomère de formule (I) (selon le protocole b) avec R qui est le résultat de la condensation entre le méthacrylate d'éthylène glycol et le toluène diisocyanate, m = p = 0, q = 1, n = 25 et R est un groupement tristyryl phenyl contenant 30 atomes de carbone.

L'essai 5 met en oeuvre un polymère hydrosoluble associatif, **caractérisé en ce qu**'il est obtenu par réaction (selon le protocole B) entre :
- 40,3 % en poids d'acide méthacrylique,
- 54,7 % en poids d'acrylate d'éthyle,
- 8,0 % en poids d'un monomère de formule (I) (selon le protocole b) dans lequel on pèse 190,1 grammes de nonylphénol ayant 15 atomes de carbone et condensé avec 50 moles d'oxyde d'éthylène) avec R qui est le résultat de la condensation entre le méthacrylate d'éthylène glycol et le toluène diisocyanate, m = p = 0, q = 1, n = 50 et R est un groupement nonyl phénol contenant 15 atomes de carbone.

L'essai 6 met en oeuvre un polymère hydrosoluble associatif, **caractérisé en ce qu**'il est obtenu par réaction (selon le protocole A) entre :
- 37,3 % en poids d'acide méthacrylique,
- 54,8 % en poids d'acrylate d'éthyle,
- 7,9 % en poids d'un monomère de formule (I) (selon le protocole a) dans lequel on pèse 439,8 grammes de l'alcool ramifié ayant 32 atomes de carbone et condensé avec 50 moles d'oxyde d'éthylène) avec R qui est le méthacrylate, m = p = 0, q = 1, n = 25 et R est un groupement alkyle ramifié contenant 32 atomes de carbone.

L'essai 7 met en oeuvre un polymère hydrosoluble associatif, **caractérisé en ce qu**'il est obtenu par réaction (selon le protocole A) entre :
- 37,4 % en poids d'acide méthacrylique,
- 55,0 % en poids d'acrylate d'éthyle,
- 7,6 % en poids d'un monomère de formule (I) (selon le protocole a) dans lequel on pèse 627 grammes l'alcool ramifié ayant 32 atomes de carbone et condensé avec 40 moles d'oxyde d'éthylène) avec R qui est le méthacrylate, m = p = 0, q = 1, n = 40 et R est un groupement alkyle ramifié contenant 32 atomes de carbone.

L'essai 8 met en oeuvre un polymère hydrosoluble associatif, **caractérisé en ce qu**'il est obtenu par réaction (selon le protocole A) entre :
- 34,6 % en poids d'acide méthacrylique,
- 57,7 % en poids d'acrylate d'éthyle,
- 7,7 % en poids d'un monomère de formule (I) (selon le protocole c) avec R qui est l'hémimaléate, m = p = 0, q = 1, n = 25 et R est un groupement alkyle ramifié contenant 32 atomes de carbone.

L'essai 9 met en oeuvre un copolymère hydrosoluble associatif, **caractérisé en ce qu**'il est obtenu par réaction (selon le protocole A) entre :
- 37,4 % en poids d'acide méthacrylique,
- 54,8 % en poids d'acrylate d'éthyle,
- 8,3 % en poids d'un monomère de formule (I) (selon le protocole a) dans lequel on pèse 376,1 grammes de l'alcool ramifié ayant 16 atomes de carbone et condensé avec 25 moles d'oxyde d'éthylène) avec R qui est le méthacrylate, m = p = 0, q = 1, n = 25 et R est un groupement alkyle ramifié contenant 16 atomes de carbone.

L'essai 10 met en oeuvre un polymère hydrosoluble associatif, **caractérisé en ce qu**'il est obtenu par réaction (selon le protocole C) entre :
- 40,3 % en poids d'acide méthacrylique,
- 54,7 % en poids d'acrylate d'éthyle,
- 5,0 % en poids d'un monomère de formule (I) (selon le protocole b) dans lequel on pèse 190,1 grammes de nonylphénol ayant 15 atomes de carbone et condensé avec 50 moles d'oxyde d'éthylène) avec R qui est le résultat de la condensation entre le méthacrylate d'éthylène glycol et le toluène diisocyanate, m = p = 0, q = 1, n = 50 et R est le groupement nonyl phenyl contenant 15 atomes de carbone.

L'essai 11 met en oeuvre un polymère hydrosoluble associatif, **caractérisé en ce qu**'il est obtenu par réaction (selon le protocole A) entre :
- 36,05 % en poids d'acide méthacrylique,
- 53,65 % en poids d'acrylate d'éthyle,
- 10,3 % en poids d'un monomère de formule (I) (selon le protocole a) dans lequel on pèse 389,8 grammes de l'alcool ramifié ayant 20 atomes de carbone et condensé avec 25 moles d'oxyde d'éthylène) avec R qui est le méthacrylate, m = p = 0, q =1, n = 25 et R est un groupement alkyle ramifié contenant 20 atomes de carbone.

L'essai 12 met en oeuvre un polymère hydrosoluble associatif, **caractérisé en ce qu**'il est obtenu par réaction (selon le protocole D) entre :
- 33,7 % en poids d'acide méthacrylique,
- 59,0 % en poids d'acrylate d'éthyle,
- 7,3 % en poids d'un monomère de formule (I) (selon le protocole a) dans lequel on pèse 376,1 grammes de l'alcool ramifié ayant 16 atomes de carbone et condensé avec 25 moles d'oxyde d'éthylène) avec R qui est le méthacrylate, m = p = 0, q = 1, n = 25 et R est un groupement alkyle ramifié contenant 16 atomes de carbone.

Seuls les essais 3 à 12 qui illustrent l'invention, et qui correspondent au choix particulier du monomère (I), ont conduit à l'obtention de solutions : on est ici parvenu à encapsuler l'oxaliplatine.

### Exemple 4 : fabrication de granulés à base de polymères hydrosolubles associatifs et d'oxaliplatine :

Dans cet exemple, on a repris certaines des formulations aqueuses qui ont permis d'obtenir une solution dans l'exemple 3 (on a conservé le même numéro d'essai).

Le pH desdites formulations a été abaissé jusqu'à 5,8 par ajout d'une solution d'acide phosphorique à 4 %.

On obtient une dispersion de particules solides dans l'eau dont la taille a été déterminée par diffusion dynamique de la lumière à l'aide d'un Zétasizer™ nano S90 commercialisé par la société MALVERN™ (tableau 2).

**Tableau 2**

| Essai n° | Taille des particules (nm) |
|---|---|
| 3 | 100 |
| 5 | 30 |
| 8 | 150 |
| 9 | 110 |
| 10 | 130 |
| 11 | 115 |

Ces dispersions ont été stockées 21 jours à température ambiante et sont stables, il n'est observé aucune sédimentation ni ré agglomération.

Elles sont finalement séchées pendant 1 heure à 110°C dans une étuve, ce qui conduit à l'obtention de granulés qui renferment de l'oxaliplatine, encapsulé dans des particules de polymère.

## Revendications

1. Formulations aqueuses, contenant au moins un composé organoplatinique et au moins un polymère hydrosoluble associatif, **caractérisées en ce que** ledit polymère est le résultat de la polymérisation :
- de l'acide (méth)acrylique,
- d'au moins un monomère non hydrosoluble, qui est préférentiellement un ester (méth)acrylique choisi très préférentiellement parmi l'acrylate d'éthyle, l'acrylate de butyle, le méthacrylate de méthyle et leurs mélanges,
- et d'au moins un monomère de formule (I) : 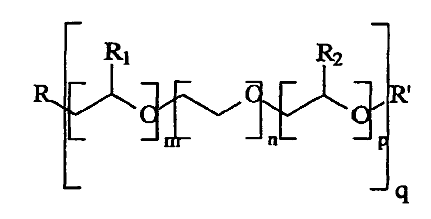
- m, n, p et q sont des entiers et m, n, p sont inférieurs à 150 et l'un d'eux au moins est supérieur à 0, préférentiellement 20,
- R comporte une fonction vinylique polymérisable,
- R₁ et R₂ sont identiques ou différents et représentent des atomes d'hydrogène ou des groupements alkyls ayant de 1 à 4 atomes de carbone,
- R' est un groupement hydrophobe ayant de 14 inclus à 32 inclus atomes de carbone, et est préférentiellement choisi parmi les groupements alkyles ramifiés ayant de 14 inclus à 32 inclus atomes de carbone, et parmi les groupements aromatiques substitués ayant de 14 inclus à 32 inclus atomes de carbone.

2. Formulations aqueuses selon la revendication 1, **caractérisées en ce que** leur pH est supérieur à 6, préférentiellement 6,5, très préférentiellement 7.

3. Formulations aqueuses selon la revendication 1, **caractérisées en ce que** leur pH est inférieur à 6, préférentiellement 3.

4. Formulations aqueuses selon l'une des revendications 1 à 3, **caractérisées en ce qu'**elles contiennent de 0,1 % à 30 %, préférentiellement de 5 % à 30 %, très préférentiellement de 10 % à 30 % en poids sec dudit composé organoplatinique, par rapport au poids sec dudit polymère hydrosoluble associatif.

5. Formulations aqueuses selon l'une des revendications 1 à 4, **caractérisées en ce qu'**elles contiennent de 0,1 % à 15 %, préférentiellement de 1 % à 10 % en poids de matière sèche par rapport à leur poids total.

6. Formulations aqueuses selon l'une des revendications 1 à 5, **caractérisées en ce que** le composé organoplatinique est choisi parmi, le carboplatine, l'oxaliplatine et leurs mélanges, et est préférentiellement l'oxaliplatine.

7. Formulations aqueuses selon l'une des revendications 1 à 6, **caractérisées en ce qu'**elles contiennent éventuellement au moins un autre agent anticancéreux choisi parmi le fluoro-uracil, le S1, l'association de la vinblastine avec la bléomycine, l'association de l'étoposide avec la bléomycine ou le paclitaxel.

8. Granulés contenant au moins un composé organoplatinique et au moins un polymère hydrosoluble associatif, **caractérisés en ce que** ledit polymère est le résultat de la polymérisation :
- de l'acide (méth)acrylique,
- d'au moins un monomère non hydrosoluble, qui est préférentiellement un ester (méth)acrylique choisi très préférentiellement parmi l'acrylate d'éthyle, l'acrylate de butyle, le méthacrylate de méthyle et leurs mélanges,
- et d'au moins un monomère de formule (I) : 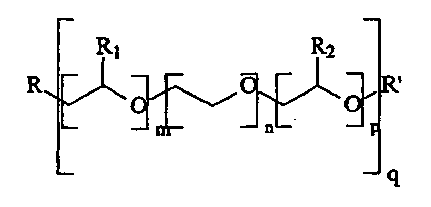
- m, n, p et q sont des entiers et m, n, p sont inférieurs à 150 et l'un d'eux au moins est supérieur à 0, préférentiellement 20,
- R comporte une fonction vinylique polymérisable,
- R₁ et R₂ sont identiques ou différents et représentent des atomes d'hydrogène ou des groupements alkyls ayant de 1 à 4 atomes de carbone,
- R' est un groupement hydrophobe ayant de 14 inclus à 32 inclus atomes de carbone, et est préférentiellement choisi parmi les groupements alkyles ramifiés ayant de 14 inclus à 32 inclus atomes de carbone, et parmi les groupements aromatiques substitués ayant de 14 inclus à 32 inclus atomes de carbone.

9. Granulés selon la revendication 8, **caractérisés en ce qu'**ils contiennent moins de 5 %, préférentiellement 1 %, très préférentiellement 0,1 % en poids d'eau, tel que mesuré par pesée différentielle après évaporation dans une étuve à 110°C pendant 1 heure.

10. Granulés selon l'une des revendications 8 ou 9, **caractérisés en ce qu'**ils contiennent de 0,1 % à 30 %, préférentiellement de 5 % à 30 %, très préférentiellement de 10 % à 30 % en poids sec dudit composé organoplatinique, par rapport au poids sec dudit polymère hydrosoluble associatif.

11. Granulés selon l'une des revendications 8 à 10, **caractérisés en ce que** le composé organoplatinique est choisi parmi le carboplatine, l'oxaliplatine et leurs mélanges, et est préférentiellement l'oxaliplatine.

12. Granulés selon l'une des revendications 8 à 11, **caractérisés en ce qu'**ils contiennent éventuellement au moins un autre agent anticancéreux choisi parmi le fluoro-uracil, le S1, l'association de la vinblastine avec la bléomycine, l'association de l'étoposide avec la bléomycine ou le paclitaxel.

13. Granulés selon l'une des revendications 8 à 12, **caractérisés en ce qu'**ils contiennent un agent d'enrobage qui est choisi parmi un polymère cellulosique, un copolymère de l'acide (méth)acrylique avec un ester acrylique, et leurs mélanges.

14. Granulés selon l'une des revendications 8 à 13, **caractérisés en ce qu'**ils contiennent un agent lubrifiant qui est préférentiellement le talc.

15. Granulés selon l'une des revendications 8 à 14, **caractérisés en ce qu'**ils contiennent un agent plastifiant qui est préférentiellement le triéthyle citrate.

16. Granulés selon l'une des revendications 8 à 15, **caractérisés en ce qu'**ils contiennent un agent tensioactif qui est préférentiellement le polysorbate 80.

17. Préparation pharmaceutique **caractérisée en ce qu'**elle contient une formulation aqueuse selon l'une des revendications 1 à 7 ou un granulé selon l'une des revendications 8 à 16.

18. Utilisation d'une formulation aqueuse selon l'une des revendications 1 à 7 ou d'un granulé selon l'une des revendications 8 à 16, pour fabriquer un médicament à administration par voie orale, et destiné à être mis en oeuvre en polychimiothérapie.

## Claims

1. Aqueous formulations, containing at least one organoplatinic compound, at least one associative water-soluble polymer, and **characterised in that** said polymer is result of the polymerization:
- of (meth)acrylic acid,
- of at least one non-water-soluble monomer, which is preferentially a (meth)acrylic ester chosen very preferentially from among ethyl acrylate, butyl acrylate, methyl methacrylate, and mixtures thereof,
- and of at least one monomer, of formula (I): 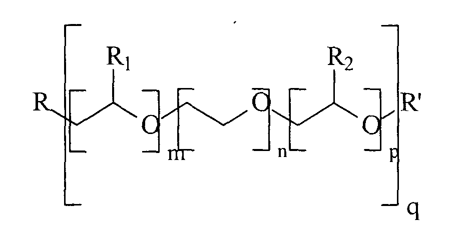
- m, n, p and q are whole numbers and m, n, p are less than 150 and at least one of them is greater than 0, preferentially 20,
- R has a polymerizable vinyl function,
- R₁ and R₂ are identical or different, and represent hydrogen atoms or alkyl groups having 1 to 4 carbon atoms,
- R' is a hydrophobic group having 14 inclusive to 32 inclusive carbon atoms, and is preferentially chosen from among branched alkyl groups having 14 inclusive to 32 inclusive carbon atoms, and from among substituted aromatic groups having from 14 inclusive to 32 inclusive carbon atoms.

2. Aqueous formulations according to claim 1, **characterised in that** their pH is greater than 6, preferentially 6.5, and very preferentially 7.

3. Aqueous formulations according to claim 1, **characterised in that** their pH is less than 6, preferentially 3.

4. Aqueous formulations according to one of the claims 1 to 3, **characterised in that** they contain 0.1% to 30%, preferentially 5% to 30%, and very preferentially 10% to 30%, by dry weight of said organoplatinic compound, relative to the dry weight of the associative water-soluble polymer.

5. Aqueous formulations according to one of the claims 1 to 4, **characterised in that** they contain 0.1 to 15%, preferentially 1% to 10% by weight of solids content in relation to their total weight.

6. Aqueous formulations according to one of the claims 1 to 5, **characterised in that** the organoplatinic compound is chosen from among carboplatin, oxaliplatin and mixtures thereof, and is preferentially oxaliplatin.

7. Aqueous formulations according to one of the claims 1 to 6, **characterised in that** they potentially contain at least one other anticancer agent chosen from among fluoro-uracil, S1, the association of vinblastine with bleomycin, the association of etoposide with bleomycin, or paclitaxel.

8. Granulates, containing at least one organoplatinic compound at least one associative water-soluble polymer, **characterised in that** said polymer is result of the polymerization:
- of (meth)acrylic acid,
- of at least one non-water-soluble monomer, which is preferentially a (meth)acrylic ester chosen very preferentially from among ethyl acrylate, butyl acrylate, methyl methacrylate, and mixtures thereof,
- and of at least one monomer, of formula (I): 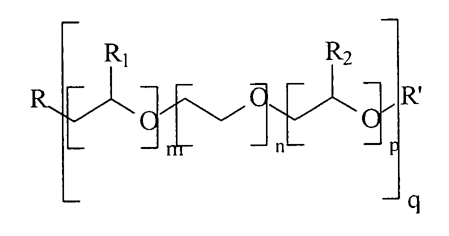
- m, n, p and q are whole numbers and m, n, p are less than 150 and at least one of them is greater than 0, preferentially 20,
- R has a polymerizable vinyl function,
- R₁ and R₂ are identical or different, and represent hydrogen atoms or alkyl groups having 1 to 4 carbon atoms,
- R' is a hydrophobic group having 14 inclusive to 32 inclusive carbon atoms, and is preferentially chosen from among branched alkyl groups having 14 inclusive to 32 inclusive carbon atoms, and from among substituted aromatic groups having from 14 inclusive to 32 inclusive carbon atoms.

9. Granulates according to claim 8, **characterised in that** they contain less than 5%, preferentially 1%, and a very preferentially 0.1% by weight of water, as measured by a differential scale after evaporation in an oven at 110°C for 1 hour.

10. Granulates according to claim 8 or 9, **characterised in that** they contain 0.1 % to 30%, preferentially 5% to 30%, and very preferentially 10% to 30%, by dry weight of said organoplatinic compound, relative to the dry weight of the associative water-soluble polymer.

11. Granulates according to one of the claims 8 to 10, **characterised in that** the organoplatinic compound is chosen from among carboplatin, oxaliplatin and mixtures thereof, and is preferentially oxaliplatin.

12. Granulates according to one of the claims 8 to 11, **characterised in that** they potentially contain at least one other anticancer agent chosen from among fluoro-uracil, S1, the association of vinblastine with bleomycin, the association of etoposide with bleomycin, or paclitaxel.

13. Granulates according to one of the claims 8 to 12, **characterised in that** they contain a coating agent which is chosen from among a cellulosic polymer, a copolymer of (meth)acrylic acid with an acrylic ester, and mixtures thereof.

14. Granulates according to one of the claims 8 to 13, **characterised in that** they contain a lubricating agent which is preferentially talc.

15. Granulates according to one of the claims 8 to 14, **characterised in that** they contain a plastifying agent which is preferentially triethyl citrate.

16. Granulates according to one of the claims 8 to 15, **characterised in that** they contain a surface-active agent which is preferentially polysorbate 80.

17. A pharmaceutical preparation **characterised in that** it contains an aqueous formulation according to one of the claims 1 to 7 or a granulate according to one of the claims 8 to 16.

18. The use of an aqueous formulation according to one of the claims 1 to 7 or a granulate according to one of the claims 8 to 16, to manufacture an orally administered medication intended to be used in polychemotherapy.

## Patentansprüche

1. Wässrige Formulierungen, die mindestens eine Organoplatinverbindung und mindestens ein wasserlösliches assoziatives Polymer enthalten, **dadurch gekennzeichnet, dass** das besagte Polymer das Ergebnis der Polymerisation:
- Der (Meth)acrylsäure,
- mindestens eines nicht wasserlöslichen Monomers, welches vorzugsweise ein (Meth)acrylester ist, vorzugsweise gewählt unter dem Ethylacrylat, dem Butylacrylat, dem Methylmethacrylat und Gemischen daraus,
- und mindestens eines Monomers gemäß der Formel (I): 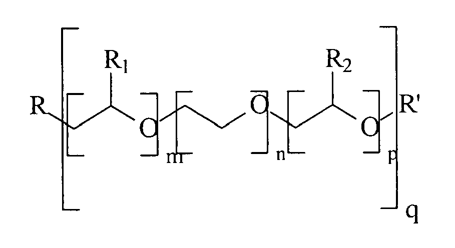 wobei
- m, n, p und q ganze Zahlen sind und m, n, p kleiner als 150 sind, und von denen mindestens eine größer als 0, vorzugsweise 20, ist,
- R eine polymerisierbare Vinylfunktion aufweist,
- R₁ und R₂ identisch oder verschieden sind und Wasserstoffatome oder Alkylgruppen mit 1 bis 4 Kohlenstoffatomen darstellen,
- R' eine hydrophobe Gruppe mit einschließlich 14 bis einschließlich 32 Kohlenstoffatomen ist und vorzugsweise unter den verzweigten Alkylgruppen mit einschließlich 14 bis einschließlich 32 Kohlenstoffatomen, und unter den substituierten aromatischen Gruppen mit einschließlich 14 bis einschließlich 32 Kohlenstoffatomen gewählt ist,
ist.

2. Wässrige Formulierungen nach Anspruch 1, **dadurch gekennzeichnet, dass** ihr pH-Wert größer als 6, vorzugsweise 6,5, in sehr bevorzugter Weise 7 ist.

3. Wässrige Formulierungen nach Anspruch 1, **dadurch gekennzeichnet, dass** ihr pH-Wert kleiner als 6, vorzugsweise 3 ist.

4. Wässrige Formulierungen nach einem der Ansprüche 1 bis 3, **dadurch gekennzeichnet, dass** sie 0,1 bis 30, vorzugsweise 5 bis 30, in sehr bevorzugter Weise 10 bis 30 Gewichtsprozent der besagten Organoplatinverbindung im Verhältnis zum Trockengewicht des besagten wasserlöslichen assoziativen Polymers enthalten.

5. Wässrige Formulierungen nach einem der Ansprüche 1 bis 4, **dadurch gekennzeichnet, dass** sie 0,1 bis 15, vorzugsweise 1 bis 10 Gewichtsprozent Trockenmasse im Verhältnis zu ihrem Gesamtgewicht enthalten.

6. Wässrige Formulierungen nach einem der Ansprüche 1 bis 5, **dadurch gekennzeichnet, dass** die Organoplatinverbindung unter dem Carboplatin, dem Oxaliplatin und Gemischen daraus gewählt und vorzugsweise das Oxaliplatin ist.

7. Wässrige Formulierungen nach einem der Ansprüche 1 bis 6, **dadurch gekennzeichnet, dass** sie gegebenenfalls mindestens einen anderen Antikrebswirkstoff, gewählt unter dem Fluoro-Uracil, dem S1, der Verbindung des Vinblastin mit dem Bleomycin, der Verbindung des Etoposid mit dem Bleomycin oder dem Paclitaxel, enthalten.

8. Granulate, die mindestens eine Organoplatinverbindung und mindestens ein wasserlösliches assoziatives Polymer enthalten, **dadurch gekennzeichnet, dass** das besagte Polymer das Ergebnis der Polymerisation:
- Der (Meth)acrylsäure,
- mindestens eines nicht wasserlöslichen Monomers, welches vorzugsweise ein (Meth)acrylester ist, vorzugsweise gewählt unter dem Ethylacrylat, dem Butylacrylat, dem Methylmethacrylat und Gemischen daraus,
- und mindestens eines Monomers gemäß der Formel (I): 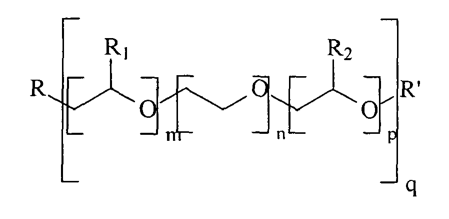 wobei
- m, n, p und q ganze Zahlen sind und m, n, p kleiner als 150 sind, und von denen mindestens eine größer als 0, vorzugsweise 20, ist,
- R eine polymerisierbare Vinylfunktion aufweist,
- R₁ und R₂ identisch oder verschieden sind und Wasserstoffatome oder Alkylgruppen mit 1 bis 4 Kohlenstoffatomen darstellen,
- R' eine hydrophobe Gruppe mit einschließlich 14 bis einschließlich 32 Kohlenstoffatomen ist und vorzugsweise unter den verzweigten Alkylgruppen mit einschließlich 14 bis einschließlich 32 Kohlenstoffatomen, und unter den substituierten aromatischen Gruppen mit einschließlich 14 bis einschließlich 32 Kohlenstoffatomen gewählt ist,
ist.

9. Granulate nach Anspruch 8, **dadurch gekennzeichnet, dass** sie weniger als 5, vorzugsweise 1, in sehr bevorzugter Weise 0,1 Gewichtsprozent Wasser, wie im Rahmen einer Differentialwägung nach Verdampfung während 1 Stunde in einem Trockenofen bei 110°C gemessen, enthalten.

10. Granulate nach einem der Ansprüche 8 oder 9, **dadurch gekennzeichnet, dass** sie 0,1 bis 30 %, vorzugsweise 5 bis 30, in sehr bevorzugter Weise 10 bis 30 Trockengewichtsprozent der besagten Organoplatinverbindung im Verhältnis zum Trockengewicht des besagten wasserlöslichen assoziativen Polymers enthalten.

11. Granulate nach einem der Ansprüche 8 bis 10, **dadurch gekennzeichnet, dass** die Organoplatinverbindung unter dem Carboplatin, dem Oxaliplatin und Gemischen daraus, gewählt und vorzugsweise das Oxaliplatin ist.

12. Granulate nach einem der Ansprüche 8 bis 11, **dadurch gekennzeichnet, dass** sie gegebenenfalls mindestens einen anderen Antikrebswirkstoff, gewählt unter dem Fluoro-Uracil, dem S1, der Verbindung des Vinblastin mit dem Bleomycin, der Verbindung des Etoposid mit dem Bleomycin oder dem Paclitaxel, enthalten.

13. Granulate nach einem der Ansprüche 8 bis 12, **dadurch gekennzeichnet, dass** sie ein Umhüllungsmittel, gewählt unter einem Cellulose-Polymer, einem Copolymer der (Meth)acrylsäure mit einem Acrylester und Gemischen daraus, enthalten.

14. Granulate nach einem der Ansprüche 8 bis 13, **dadurch gekennzeichnet, dass** sie ein Gleitmittel, welches vorzugsweise der Talk ist, enthalten.

15. Granulate nach einem der Ansprüche 8 bis 14, **dadurch gekennzeichnet, dass** sie einen Weichmacher, welcher vorzugsweise das Triethylcitrat ist, enthalten.

16. Granulate nach einem der Ansprüche 8 bis 15, **dadurch gekennzeichnet, dass** sie ein Tensid, welches vorzugsweise das Polysorbat 80 ist, enthalten.

17. Pharmazeutische Zubereitung, **dadurch gekennzeichnet, dass** sie eine wässrige Formulierung gemäß einem der Ansprüche 1 bis 7 oder ein Granulat gemäß einem der Ansprüche 8 bis 16 enthält.

18. Vcrwcndung einer wässrigen Formulierung gemäß einem der Ansprüche 1 bis 7 oder eines Granulats gemäß einem der Ansprüche 8 bis 16, zur Herstellung eines Arzneimittels zur oralen Verabreichung für die Anwendung in der Polychemotherapie.
